# EUROPEAN PATENT SPECIFICATION

(11) **EP 3 854 225 B1**
(45) Date of publication and mention of the grant of the patent: **09.09.2026**
(21) Application number: 18827371.8
(22) Date of filing: 19.09.2018
(51) Int. Cl.: A23L 19/00, A23L 29/206

(54) **PROPHYLACTIC AGENT FOR SPONTANEOUS CANCERS**
PROPHYLAKTISCHES MITTEL FÜR SPONTANE KREBSERKRANKUNGEN
AGENT PROPHYLACTIQUE POUR DES CANCERS SPONTANÉS

(43) Date of publication of application: 28.07.2021
(73) Proprietor: Nihon Sizen Hakkoh Co., Ltd., Takayama-shi, Gifu 501-5401 (JP)
(72) Inventor: HIGASHI, Naoki, Tokyo 145-0071 (JP); NAKANISHI, Masahiro, Takayama-shi Gifu 501-5401 (JP)
(74) Representative: Wächtershäuser & Hartz Patentanwaltspartnerschaft mbB
(86) International application number: PCT/JP2018/034617
(87) International publication number: WO 2019/009437

(56) References cited:
- EP-A1- 3 231 437
- WO-A1-02/072123
- WO-A1-2006/093267
- WO-A1-2016/093104
- CN-A- 103 027 294
- JP-A- 2002 138 046
- JP-A- 2003 033 125
- JP-A- 2004 107 286
- JP-A- 2006 117 575
- JP-A- 2007 084 504
- JP-A- 2007 230 973
- JP-A- 2007 319 157
- JP-A- 2008 255 057
- JP-A- 2013 237 652
- JP-A- 2015 202 065
- JP-A- H09 169 661
- TAKEDA TOSHIO ET AL.: "Senescence-accelerated Mouse (SAM) : With Special Reference to Development and Pathobiological Phenotypes", ILAR JOURNAL, vol. 38, no. 3, 1 July 1997 (1997-07-01), pages 109 - 118, XP055680741, ISSN: 1084-2020, DOI: 10.1093/ilar.38.3.109
- CARTER TODD A ET AL.: "Mechanisms of aging in senescence-accelerated mice", GENOME BIOLOGY, vol. 6, no. 6, 2005601, pages 1 - 17, XP021013013, ISSN: 1465-6906, DOI: 10.1186/gb-2005-6-6-r48

## Description

### Technical Field

The present invention relates to a prophylactic agent for a spontaneous cancer in which a plant fermentation product is used.

The invention is defined by the appended claims 1-5.

### Background Art

Most of the cancers produced in humans are spontaneous cancers which rapidly increase with aging and senescence. As a cause of spontaneous cancers which rapidly increase with senescence, chronic inflammation has been recognized (NPLs 1 to 4) .

Ultimate inhibition of a cancer is inhibition of production of the cancer, but a long period of time is required for evaluating the inhibition of production of a spontaneous cancer with senescence, which hinders the development. It is quite difficult in terms of not only the time but also the ethics to conduct in humans a production inhibition test of a spontaneous cancer that needs a long period of time for the production. Thus, it is possible to use rodents which have a short life span. However, the test system for evaluating cancers spontaneously produced with senescence is limited, and moreover, a time period as long as two years is required.

Although various animal model test systems have thus been designed and have increased in the effect, such systems are still insufficient for evaluating the inhibitory effect on the production of cancers spontaneously produced with senescence which account for a majority of human cancers. Accordingly, an evaluation system for spontaneous cancers using an evaluation system associated with senescence is essential for evaluating prophylaxis of cancers (NPL 5).

The present inventors have demonstrated, using a senescence accelerated mouse (SAM), that a plant fermentation product significantly extends the life and inhibits senescence, and have already acquired a patent as an aging inhibitor (PTL 1). The SAM used in this evaluation system is sold and supplied under the guidance of the Society for Senescence-Accelerated Mouse (SAM) Research (http://www.samrc.jp/), and the properties are reported by TAKEDA Toshio who is a discoverer and developer of the SAM (NPL 6). According to the report, the life span of the SAM is 9.7 months (38.8 weeks) which is shorter than 60 weeks in which cancer production is generally observed in a mouse, and thus the SAM has not been able to be used in an evaluation system for spontaneous cancers.

### Citation List

### Patent Literature

PTL 1: Japanese Patent No. 6013670

CN103027294 discloses the production of an anticancer enzyme comprising mixing anticancer fruit fermented raw liquid, vegetable fermented raw liquid, edible bacteria fermented raw liquid, and bean nutrition fermented raw liquid, canning and ripening to obtain the concentrated black viscous anticancer enzyme.

### Non-patent Literature

NPL 1: "Mansei Ensho to Gan no Kakawari (relation of chronic inflammation to cancer" TAKAYAMA Shozo, YASUFUKU Kazue, Modern Media, vol.51 (4) p.17-19 (2005)
NPL 2: "Saibo Roka to Mansei Ensho (cell senescence and chronic inflammation", YAMAKOSHI Kimi, Nippon Ronen Igakukai-Shi (journal of The Japan Geriatrics Society), vol.53 (2) p.88-94 (2016)
NPL 3: "Meneki Roka no Mekanizumu wo Kaimei Shimasita (we elucidated the mechanism of immunosenescence" (http://www.kyoto-u.ac.jp/static/ja/news_data/h/h1/news6/2009/090908_1.htm), Professor MINATO, Kyoto University Home Page, published on September 8, 2009
NPL 4: "Ensho wo Haikei tosita Mausu Daicyo Hatsugan Moderu niyoru Kagaku Yobo Kenkyu (study for chemoprophylaxis by a mouse large intestine carcinogenesis model against a background of inflammation", YASUI Yumiko, Journal of the Hokkaido Veterinary Medical Association, vol. 54, p.688-691 (2010)
NPL 5: "Hatsugan Moderu Mausu no Genjo to Tenbo (current situation and vision of carcinogenesis model mouse" (https://www.pmda.go.jp/files/000198150.pdf), YAO Ryoji, December 18, 2014, the 2nd special committee on application of nonclinical test, Document 3
NPL 6: "Roka Sokushin Moderu Mausu (SAM) - Roka Byotai to sono Seigyo wo Cyusin ni - (senescence accelerated model mouse (SAM) - focusing around senescence pathology and its control", TAKEDA Toshio, Nippon Eisei-gaku Zasshi (journal of The Japanese Society for Hygiene) , vol.51, p.569-578 (1996)

### Summary of Invention

### Technical Problem

Seeking to improve the evaluation ability of the SAM evaluation system, the present inventors have had an object to examine whether the SAM evaluation system can be used as an evaluation system for spontaneous cancers, and to examine, using the evaluation system, whether a plant fermentation product used also in PTL 1 has a spontaneous cancer prophylactic effect.

### Solution to Problem

The present inventors made intensive and extensive studies for achieving the above object. In the course of the study, it was found that a mass-like process was produced in the inguinal region or hypogastric region of a plurality of mice and that the production tended to be inhibited in a group in which the plant fermentation product of a high concentration was taken. All the mice were dissected and all the regions where a mass was recognized were observed, and then it was found that only a cystiform fat mass was observed but no tumor was found. It was found that only one of four normal SAM type mice that were raised as controls was able to be raised for a long period (to 157 weeks old) . It was found by autopsy that a white solid was recognized at the mesenteric lymph node of only this mouse. The shape thereof was closely resembled to that of a lymphoma shown in this description. It was also found that the SAM type mice were able to be raised for a long period of time by controlling the raising environment. It was thus found that a spontaneous cancer evaluation system can be configured with a system using SAM type mice.

The present inventors have also found by using the SAM spontaneous cancer evaluation system that a plant fermentation product which is also used in PTL 1 has a spontaneous cancer prophylactic effect.

Specifically, the present invention relates to a prophylactic agent for a spontaneous cancer according to claim 1.

The present invention relates to the prophylactic agent for a spontaneous cancer, the prophylactic agent further containing a therapeutic drug for a cancer and/or an alternative therapeutic drug for a cancer.

The present invention relates to the prophylactic agent for a spontaneous cancer, in the form of a food or a drink.

### Advantageous Effects of Invention

The present invention has obviously demonstrated that a certain plant fermentation product has an inhibitory effect on the production of a spontaneous cancer. This is the first demonstration for a plant fermentation product.

The spontaneous cancer mouse of the present disclosure, but not part of the invention, can be raised for a long period of time to 70 or more weeks old, and thus can be used for evaluation of an effect on cancers spontaneously produced with senescence, such cancers accounting for a majority of human cancers.

In addition, the mouse can reduce the test period until 60 weeks old during which a spontaneous cancer is frequently produced to 46 weeks even if the test start time is delayed until 14 weeks old. Thus, a test period closed to 36 weeks old (the test period is 30 weeks) which is the test period for a genetically modified rasH2 mouse can be achieved, and therefore the mouse is useful.

### Brief Description of Drawings

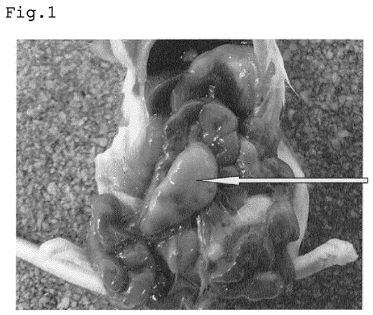

Fig. 1: It shows a tumor at the mesenteric region of an SAMP1 mouse (male) (the arrow indicates a lymphoma).

### Description of Embodiments

The prophylactic agent for a spontaneous cancer of the present invention contains a plant fermentation product described below as an active ingredient. The plant fermentation product is a mixture of the plant fermentation products of the following (a) to (g).

(a) A fermentation product obtained by allowing a koji mold to act on one or two or more beans or grains selected from the group consisting of barley, black soybean, red rice, black rice, adzuki bean, adlay, Japanese barnyard millet, foxtail millet, and proso millet.
(b) A fermentation product obtained by allowing a yeast to act on one or two or more fruits selected from the group consisting of mandarin orange, grape, apple, Vitis Coignetiae berry, peach, persimmon, papaya, Japanese pear, watermelon, Japanese apricot, fig, Chinese quince fruit, kabocha squash, kumquat, Yuzu, loquat fruit, apricot, jujube, chestnut, matatabi fruit, and Japanese plum.
(c) A fermentation product obtained by allowing a lactic acid bacterium to act on one or two or more root vegetables or potatoes selected from the group consisting of purple sweet potato, Jerusalem artichoke, carrot, onion, sweet potato, taro, Japanese wild yam, daikon, red turnip, great burdock, lotus root, yacon, lily bulb, arrowhead, ginger, garlic, and turmeric.
(d) A fermentation product obtained by allowing a lactic acid bacterium to act on one or two or more flowers or leafy vegetables selected from the group consisting of cabbage, perilla, Morus leaf, fish mint, Japanese mugwort, Sasa veitchii, and dandelion.
(e) A fermentation product obtained by allowing a yeast to act on one or two or more seaweeds selected from the group consisting of kelp, wakame, and mozuku.
(f) A fermentation product obtained by fermenting, with a yeast one or two or more seeds selected from the group consisting of black sesame, walnut, and ginkgo nuts.
(g) A fermentation product obtained by allowing lactic acid bacterium to act on one or two or more mushrooms selected from the group consisting of maitake and shiitake
   Whereby the plant fermentation product is obtainable or obtained by a process comprising the following steps:
   (i) inoculating a culture fluid prepared so as to contain a lactic acid bacterium of P. acidilacti, L. brevis, L. mesenteroides, L. plantarum, L. lactis, L. sakei, and L. casei at a bacterial concentration of about 1.0 × 105 cfu/g at 0.2% by mass into raw materials of the above (c), (d), and (g), followed by culture at 30°C for 50 hours;
   (ii) inoculating a culture fluid prepared so as to contain a yeast of S. cervisiae and Z. rouxii at a bacterial concentration of about 1.0 × 105 cfu/g at 0.4% by mass into raw materials of the above (b), (e), and (f), followed by culture at 30°C for 50 hours;
   (iii) inoculating a koji mold of yellow koji mold, black koji mold, and white koji mold at 0.1% by mass into a raw material of (a), followed by culture at 35°C for 70 hours;
   (iv) mixing each culture, followed by culture at 30°C for 200 hours;
   (v) subjecting the mash remaining after the fermentation to a solid-liquid separation operation,
   (vi) concentrating the resulting filtrate into a paste,
   (vii) dispensing the paste into containers, followed by post-fermentation for further one year to obtain the plant fermentation product. .

A plant, such as a bean or grain or a fruit, to be subjected to the fermentation may be used as it is, or may be subjected to a pretreatment, such as pulverization or drying, as required. The plant may be diluted with water added, as required.

Through the maturing process by the post-fermentation, the taste and the formulation capability can be improved and the antioxidative activity can also be increased.

The plant fermentation product as an active ingredient used in the present invention has properties of the following (1) to (4).

### (1) Taste

The plant fermentation product has sweetness due to a fruit, a vegetable, or another raw material and sweetness due to a koji mold as well as an organic acid. The plant fermentation product contains a polyphenol derived from a raw material but has less bitterness.

### (2) Solubility in water

The plant fermentation product is easily dissolved in water.

### (3) Stability

The plant fermentation product is stable against heat and acids and a paste thereof does not putrefy and is not changed in the taste even after preservation at room temperature for one year.

### (4) Safety

The vegetable, fruit, herb, and the like used as a raw material, which are ordinary foods, and the yeast, lactic acid bacterium, and koji mold used in the fermentation, which are a microorganism derived from brewage of a food or from a pickle or the like, have lots of experiences as food.

The plant fermentation product used in the present invention has properties of the following (i) to (iv).

(i) Common components (based on 100 g)

| | |
|---|---|
| Water | 15 to 35 g |
| Protein | 5 to 20 g |
| Lipid | 1 to 8 g |
| Ash | 1 to 5 g |
| Carbohydrate | 30 to 70 g |
| Sodium | 40 to 150 mg |
| Vitamin B6 | 0.1 to 0.5 mg |
| Energy | 200 to 500 kcal |

(ii) Amino acid composition (based on 100 g)

| | |
|---|---|
| Arginine | 0.2 to 0.6 g |
| Lysine | 0.1 to 0.7 g |
| Histidine | 0.1 to 0.4 g |
| Phenylalanine | 0.2 to 0.8 g |
| Tyrosine | 0.1 to 0.6 g |
| Leucine | 0.3 to 1.2 g |
| Isoleucine | 0.2 to 0.8 g |
| Methionine | 0.05 to 0.3 g |
| Valine | 0.2 to 0.9 g |
| Alanine | 0.2 to 0.9 g |
| Glycine | 0.2 to 0.7 g |
| Proline | 0.4 to 1.2 g |
| Glutamic acid | 1.2 to 3.0 g |
| Serine | 0.2 to 0.8 g |
| Threonine | 0.2 to 0.7 g |
| Aspartic acid | 0.4 to 1.5 g |
| Tryptophan | 0.03 to 0.15 g |
| Cystine | 0.05 to 0.40 g |

(iii) Organic acid composition (based on 100 g)

| | |
|---|---|
| Citric acid | 0.5 to 1.2 g |
| Malic acid | 0.05 to 0.5 g |
| Succinic acid | 0.04 to 0.3 g |
| Lactic acid | 0.5 to 6.0 g |
| Formic acid | 0.01 to 0.1 g |
| Pyruvic acid | 0.005 to 0.05 g |
| Free γ-aminobutyric acid | 0.01 to 0.05 g |

(iv) Mineral composition (based on 100 g)

| | |
|---|---|
| Phosphorus | 100 to 400 mg |
| Iron | 1 to 5 mg |
| Calcium | 500 to 900 mg |
| Potassium | 600 to 1000 mg |
| Magnesium | 70 to 120 mg |
| Zinc | 0.8 to 1.6 mg |
| Iodine | 1.0 to 2.5 mg |

The prophylactic agent for a spontaneous cancer of the present invention is obtained by adding a pharmaceutically acceptable carrier, excipient, water activity modifier, or the like to the thus-obtained plant fermentation product to make a formulation according to a known pharmaceutical production method. As required, the plant fermentation product may be concentrated to adjust the concentration, and may be made into powder by spray drying or freeze drying. Examples of the carrier or excipient used in the formulation include lactose, glucose, sucrose, starch, and a sugar mixture. Examples of the final form for use include solution, paste, soft capsule, chewable, and capsule. In an example of the dosage and usage, an adult orally takes about 0.1 g or more, preferably 0.1 to 12 g, more preferably 0.6 to 10 g (in terms of the solid) of the plant fermentation product as an active ingredient a day.

The prophylactic agent for a spontaneous cancer of the present invention can be made into the form of medicine, quasi drug, or the like, or can be made into the form of food or drink by formulating a known food material therewith. The plant fermentation product can be taken as it is, but may be filtered and concentrated after sterilization for increasing the storability, or, as required, may be made into powder by adding an excipient, followed by spray drying or freeze drying. Furthermore, for increasing the shelf life during distribution, the prophylactic agent is preferably concentrated to reduce the water activity. Examples of the form of food or drink include paste, soft capsule, tablet, and drink. When an adult orally takes about 0.1 g or more, preferably 0.1 to 12 g, more preferably 0.6 to 10 g (in terms of solid) of the plant fermentation product a day, an excellent spontaneous cancer prophylactic effect or the like can be achieved. Examples of commercially available products in which such a plant fermentation product is made into a formulation in the form of a food include Amo Koso (fermented botanical food) Kin-jirushi (gold label) and Amo Koso capsule (manufactured by Nihon Shizen Hakko Co. Ltd).

Besides the above products, the food or drink containing the prophylactic agent for a spontaneous cancer of the present invention may be a food or drink that contains the plant fermentation product which is an active ingredient as one of seasonings and the like, examples of the food or drink including a seasoning, such as Miso; bread, such as pan loaf; sweets, such as rice crackers, cookies, chocolates, candies, sweet buns, and cakes; dairy products, such as yogurt and cheese; pickles, such as Takuan; noodles, such as Soba and Udon; soups, such as corn potage and Wakame soup; beverages, health drinks, carbonated drinks, and fruit juice drinks.

Furthermore, a therapeutic drug for a cancer and/or an alternative therapeutic drug for a cancer may further be incorporated into the prophylactic agent for a spontaneous cancer of the present invention to enhance the effect. The therapeutic drug for a cancer is not particularly limited, and examples thereof include a cytotoxic anticancer agent, such as an antimetabolite, an alkylation agent, an anticancer antibiotic, or a microtubule inhibitor, a molecular target therapeutic drug, a platinum-based drug, and a topoisomerase inhibitor. The alternative therapeutic drug for a cancer is not particularly limited, and examples thereof include an herbal medicine, such as juzen-taiho-to, dai-saiko-to, or hochu-ekki-to, fucoidan, phellinus linteus, plant worm, Panax notoginseng, Deer horn shape ganoderma lucidum, Hedyotis diffusa, and fermentation agaricus. When the therapeutic drug for a cancer and/or the alternative therapeutic drug for a cancer is incorporated in the prophylactic agent for a spontaneous cancer of the present invention, the content thereof may be appropriately set according to the intended effect.

The prophylactic agent for a spontaneous cancer containing the plant fermentation product as an active ingredient has not only an action to inhibit production of a spontaneous cancer but also an action to inhibit production of a precancerous lesion.

The spontaneous cancer mouse of the present disclosure, but not part of the invention, can be obtained by raising an SAMP mouse from 6 to 14 weeks old, preferably 8 to 12 weeks old, more preferably 10 weeks old to 60 or more weeks old, preferably to 70 weeks old while maintaining SPF conditions. An example of the SAMP mouse is SAMP1/SkuSlc (commercially available from Sankyo Labo Service Corporation). The "while maintaining SPF conditions" means further strengthening the environment cleaning method for maintaining the SPF. In general, although SPF conditions are mentioned, actual SPF conditions are rarely maintained in a precise sense.

Here, the "SPF conditions" means conditions that are commonly known in operations in animal experiment laboratories. For example, a raiser changes his/her clothes and shoes to a sterilized laboratory coat and room shoes and wears surgical gloves and a surgical cap in a front room equipped with a UV irradiator. The facility for raising, for example, has a front room (sterilized with a UV light) for the barrier between an animal raising room and a corridor and for change of clothes, and further has an air conditioner equipped with a differential pressure gauge and filters, such as an outlet filter and an HEPA filter. Such an air conditioning system that gives a difference between the inner pressure of the front room and the inner pressure of the animal raising room to prevent the animal room from contamination is provided. The front room and the animal raising room are sterilized with rubbing alcohol, dust is adsorbed with an adhesive mat, and a front mat and feed are stored in the front room until use.

Examples of environment cleaning methods include increasing the frequency of replacement of the outlet filter of the air conditioner, installing an air cleaner with a sterilization function, such as plasmacluster, and regularly wiping the floor with an antiseptic solution, such as Hibitane. In particular, the outlet filter of the air conditioner was replaced based on an increase of 1 Pa on the differential pressure gauge in the front room or based on the discoloration of the filter due to powder on the floor mat.

When an SAMP mouse raised as above exceeds 60 weeks old, a cancer is spontaneously produced (no cancer is recognized at 56 weeks old). Whether a cancer is produced can be examined by a conventionally known method.

The use of the spontaneous cancer mouse of the present disclosure, but not part of the invention, enables evaluation of an effect of a subject substance on a spontaneous cancer. Not that the subject substance is not particularly limited.

Specifical examples of methods for evaluating an effect of a subject substance on a spontaneous cancer using the spontaneous cancer mouse include a method in which a subject substance is administered to spontaneous cancer mice and a method in which a subject substance is administered to SAMP mice during raising the SAMP mice under the above conditions.

### Examples

The present invention will be described in detail below with reference to examples, but the present invention is in no way limited to the examples.

### Production Example 1

### Production of plant fermentation product:

The following plants were used as raw materials.
(a) Bean or grain (barley, black soybean, red rice, black rice, adzuki bean, adlay, Japanese barnyard millet, foxtail millet, proso millet)
(b) Fruit (mandarin orange, grape, apple, Vitis Coignetiae berry, peach, persimmon, papaya, Japanese pear, watermelon, Japanese apricot, fig, Chinese quince fruit, kabocha squash, kumquats, Yuzu, loquat fruit, apricot, jujube, chestnut, matatabi fruit, Japanese plum)
(c) Root vegetable (purple sweet potato, Jerusalem artichoke, carrot, onion, sweet potato, taro, Japanese wild yam, daikon, red turnip, great burdock, lotus root, yacon, lily bulb, arrowhead, ginger, garlic, turmeric)
(d) Flower or leafy vegetable (cabbage, perilla, Morus leaf, fish mint, Japanese mugwort, Sasa veitchii, dandelion)
(e) Seaweed (kelp, wakame, mozuku)
(f) Seed (black sesame, walnut, ginkgo nut)
(g) Mushroom (maitake, shiitake)

Into raw materials of the above (c), (d), and (g) (730 g), a culture fluid that was prepared so as to contain a lactic acid bacterium (P. acidilacti, L. brevis, L. mesenteroides, L. plantarum, L. lactis, L. sakei, L. casei) at a bacterial concentration of about 1.0 × 10⁵ cfu/g was inoculated at 0.2% by mass, followed by culture at 30°C for 50 hours. Meanwhile, into raw materials (900 kg) of the above (b), (e), and (f), a culture fluid that was prepared so as to contain a yeast (5 kinds of S. cervisiae, 2 kinds of Z. rouxii) at a bacterial concentration of about 1.0 × 10⁵ cfu/g was inoculated at 0.4% by mass, followed by culture at 30°C for 50 hours. Into a raw material (1000 kg) of (a) a bean or grain, a koji mold (yellow koji mold, black koji mold, white koji mold) was inoculated at 0.1% by mass, followed by culture at 35°C for 70 hours. Then, each culture was mixed, followed by culture at 30°C for 200 hours. The mash remaining after the fermentation was subjected to a solid-liquid separation operation, the resulting filtrate was concentrated into a paste, which was dispensed into containers, followed by post-fermentation (maturing) for further one year to obtain a plant fermentation product.

The plant fermentation product obtained in Production Example 1 had the following properties.

### (i) General analysis values (based on 100 g)

| | |
|---|---|
| Water | 25.2 g |
| Protein | 11.8 g |
| Lipid | 3.6 g |
| Ash | 2.1 g |
| Carbohydrate | 57.3 g |
| Sodium | 54.0 mg |
| Vitamin B6 | 0.20 mg |
| Energy | 309 kcal |

### (ii) Amino acid composition (based on 100 g)

A sample was hydrolyzed with 6 N hydrochloric acid, and was then analyzed with an amino acid automatic analyzer. For cystine, after a performic acid oxidation treatment, hydrochloric acid hydrolyzation was used. For tryptophan, high performance liquid chromatography was used.

| | |
|---|---|
| Arginine | 0.33 g |
| Lysine | 0.34 g |
| Histidine | 0.22 g |
| Phenylalanine | 0.51 g |
| Tyrosine | 0.32 g |
| Leucine | 0.74 g |
| Isoleucine | 0.42 g |
| Methionine | 0.13 g |
| Valine | 0.54 g |
| Alanine | 0.48 g |
| Glycine | 0.42 g |
| Proline | 0.92 g |
| Glutamic acid | 2.25 g |
| Serine | 0.4 g |
| Threonine | 0.36 g |
| Aspartic acid | 0.84 g |
| Tryptophan | 0.06 g |
| Cystine | 0.15 g |

### (iii) Organic acid composition (based on 100 g)

| | |
|---|---|
| Citric acid | 0.81 g |
| Malic acid | 0.31 g |
| Succinic acid | 0.12 g |
| Lactic acid | 1.17 g |
| Formic acid | 0.03 g |
| Pyruvic acid | 0.01 g |
| Free γ-aminobutyric acid | 24 mg |

### (iv) Mineral composition (based on 100 g)

| | |
|---|---|
| Phosphorus | 262 mg |
| Iron | 2.65 mg |
| Calcium | 72.1 mg |
| Potassium | 798 mg |
| Magnesium | 97.8 mg |
| Zinc | 1.19 mg |
| Iodine | 1.7 mg |

### Test Example 1

Carcinogenesis inhibition test:

### (1) Mouse

SAMP1/SkuSlc (male) mice (hereinafter referred to as "SAMP1") were purchased from Sankyo Labo Service Corporation and were used. Mice of 12 weeks old were used in this test. The mice were raised in a mouse raising facility (23±2°C, humidity 50±10%, light-dark cycle of lighting from 20:00 to 8:00) according to an SPF specification. Eight to nine mice were used per group for each sample and were raised solely using a polycarbonate cage manufactured by CLEA Japan, Inc. Feed was 500 N (sterilized with γ-ray) purchased from Sankyo Labo Service Corporation and was allowed to be freely taken. As a floor mat, a high-temperature-sterilized one manufactured by CLEA Japan, Inc. was used. The cage was replaced once a week. The mice were raised until 73 weeks old.

### (2) Sample

As a sample, a plant fermentation product in a paste form produced in Production Example 1 was used. The plant fermentation product was dissolved in ion exchange water at 2.0%, and the solution was dispensed into 500-ml medium bottles, which were then all sterilized in an autoclave (121°C, 20 minutes). The supernatant was antiseptically transferred into a water supply bottle (sterilized) so that the nozzle of the water supply bottle was not clogged with generated precipitates, and was subjected to the test. Note that, with reference to a case in which a relatively larger amount of a plant fermentation product is routinely taken (77 g/week = 11 g/day) , the amount of the plant fermentation product administered was determined by calculating a value corresponding to 30 times the above amount and converting the resulting value into an amount per Kg body weight. This conversion rate is a numerical value that is empirically used in Institute of Natural Medicine in University of Toyama when an herbal medicine is applied to mice. (Matsumoto Kinzo Personal Information, The 28th SAM workshop "Roka Moderu Doubutsu SAMP8 no Ninchi Kodo Syogai ni taisuru Kanpoyaku, Cho-to-san oyobi Cyuyaku Kangenkaryu no Kaizen Koka to Sorera no Sinkeikiko (improvement effect of herbal medicine, diao-teng-san and Chinese medicine, granular Kangen on cognition activity disorder of senescence model animal SAMP8 and neural mechanism thereof", July 5 (2013), Aichi Gakuin University). When a mouse drinks about 30 ml of a 2.0% concentration sample one day, the amount corresponds to about 10 g/day for a human. The plant fermentation product diluted with ion exchange water as described above was allowed to be freely taken as drinking water.

### (3) Evaluation of cancer

### 1) Appearance, palpation

The appearance observation and palpation were performed for the presence of abnormal mass, and the appearance observation and palpation were performed for the tendency of ascites, such as abdominal bloating, and the results were recorded. Abdominal bloating was observed in one mouse. A rigid mass of about 10 mm square was produced in a femoral region in the 71th week (senescence degree = 5.5) in one example in the sample group, and the mass was rapidly bloated, but the increase rate was reduced in the 72th week. Although there was a little trouble on the action, the senescence evaluation score was not so high and a good QOL was given (senescence degree = 6.0, mainly skin appearance and curvature of backbone). The comprehensive score of the final senescence degree evaluation was 6.0, and senescence was only shown in the appearance (the gloss of hair was slightly reduced and the walking was insufficient due to the mass). Note that the senescence degree was evaluated based on the standard of the scientific society in which scoring is made based on the symptom ("The Grading Score System: A Method for Evaluating the Degree of Senescence in SAM Strains of Mice" M. Hosokawa et al, "The Senescence-accelerated Mouse (SAM) Achievements and Future Directions", Elsevier, edited by TOSHIO Takeda (2013), p.561-567).

### 2) Organs in body cavity

Somnopentyl 70 µl/100 g was intraperitoneally administered to all the mice which survived in the 73th week, and then the mice were subjected to laparotomy to observe organs in the body cavity. The incision was made to the neck to observe other organs in the body cavity, and then one white mass per mouse of approximately a little finger tip size was found in the mesenteric region in the control group (ion exchange water) (Fig. 1). In this figure, a mass with capillary invasion was observed. The histopathologic examination of the masses was entrusted to Biopathology Institute, Co., Ltd., and then the masses were determined to be B cell lymphomas. A lymphoma in a mouse corresponds to a malignant lymphoma in a human.

No tumor was observed in the other organs and the mesenteric lymph node was presumed to be primary. A mass grown into a bunch shape having a diameter of about 5 mm was also observed in an example, and this was also a B cell lymphoma. Besides, in a mouse which died in the preceding day of the anatomy, a sudden hypertrophy produced immediately before the anatomy was found in the thymus neck, and the weight was 0.6354 g. In the histopathologic examination, it was a grown mass of abnormal T lymphocyte, but whether it was malignant was not able to be determined since the sample was a postmortem one. Since an extreme atrophy due to thymus hypertrophy was found in the heart and the lung, the cause of death was determined as compression. In incision of the solid mass observed in the right femoral region of one example in the 2% group, the mass was encysted with rigid fibered interstitium in a cocoon form. When the precure was incised and extracted, an osteoid tissue was observed. By the histopathologic examination, the osteoid tissue was determined as an osteosarcoma. There is a report that in a cancer tissue, hyperfibrinolysis occurs in a microenvironment of the cancer to promote metastasis or dissemination of the cancer, whereas it is also known that interstitium rigidly surrounds a cancer tissue by the biophylaxis to inhibit the growth ("Genpatsusei Kofukumaku narabini Cyokanshuyo no Rinsyo Byorigakuteki Kento (clinicopathological study on primary retroperitoneum and mesenterium tumor)" MATSUSHITA Masahiro, et. al., Nihon Rinsyo Geka Gakkai Zassi (Journal of Japan Surgical Association) vol.47 (8), p.59-66 (1986)). In the case of the above mouse, no tumor tissue was recognized in other regions, and the senescence evaluation score was not so high, the QOL was not decreased, and thus this case was considered as an example of a biophylaxis reaction. Table 1 shows the numbers of mice in which a tumor was produced, separately for the regions inside and outside the body cavity. Table 2 shows an analysis on the average weight of tumors. Table 3 shows a list of characteristics of each tumor. Table 4 shows the numbers of abnormally grown masses.

**[Table 1]**

| Sample concentration | Number of surviving mice / number of mice at start | Average senescence score |
|---|---|---|
| 0% | 6/9 (67%) | 7.8 |
| 2% | 6/9 (67%) | 3.8 |

In Table 1, the senescence score of the 0% group was significantly higher than that of the 2% group (P = 0.0099 in t-test) . In particular, the 2% group was superior in the gloss of hair.

**[Table 2]**

| Sample concentration | Number of surviving mice | Tumor in body cavity | Tumor outside body cavity | Total |
|---|---|---|---|---|
| 0% | 6 | 3 (50%) | 1 (17%) | 4 (66%) |
| 2% | 6 | 0 (0%) | 1 (17%) | 1 (17%) |

As shown in Table 2, the frequency of production of tumor or abnormally grown mass in the mice surviving until 73 weeks old was higher in the water control group, namely the 0% group, and was 4 in 6, and the production ratio was about 66%. Note that, for a mouse that seemed to have significant ascites in the appearance, a great liver hypertrophy was observed but no ascites was observed in the autopsy. There was nothing wrong also in the color of liver, and no tumor was observed inside the section. In the water administration group, there were many mice in which a spleen hypertrophy was observed, but no tumor was found. The average survival rate at the end of the test in the 2% group was 456.25 days which was longer than 385.85 days in the 0% group, but the result was not significant according to the Kaplan-Meier analysis.

In the sample concentration 2% group, the tumor production occurred in one mouse among 6 mice, and the incidence of a lymphoma in the body cavity, which was frequently occurred in the 0% administration mice, was 0%. Thus, the plant fermentation product which was orally administered significantly inhibited the production of a spontaneous tumor. It is at least the first example that demonstrated inhibition of a spontaneous cancer by a plant fermentation product. Regarding the osteosarcoma which was produced in one example in the sample group, the entire periphery thereof was covered with a rigid membrane which was considered to be derived from interstitium representing the biophylaxis, suggesting an action of defense function. This mouse showed a low apparent senescence degree evaluation score (6.0), and the mouse seemed to coexist together with the tumor.

**[Table 3]**

| Sample concentration in drinking water | Weight of tumors in body cavity (g) | Weight of tumors outside body cavity (g) | Total weight (g) |
|---|---|---|---|
| 0% | 11.93 | 0.2 | 12.13 |
| 2% | 0 | 2.62 | 2.62 |

As is apparent from Table 3, the tumor weight in the 2% plant fermentation product administration group was lower than that of the 0% group. An inhibitory effect on the growth of a spontaneous cancer was found in the 2% plant fermentation product.

**[Table 4]**

| Sample concentration in drinking water | Number in body cavity | Number outside body cavity | Total number |
|---|---|---|---|
| 0% | 5 | 5 | 10 |
| 2% | 0 | 1 | 1 |

As shown in Table 4, the number of abnormally grown masses was 10 in the 0% plant fermentation product group, whereas the number was one in the 2% plant fermentation product group. Thus, it was apparent that the 2% plant fermentation product had an inhibitory effect on the production of a spontaneous cancer.

It was found from the above results that the 2% plant fermentation product has an inhibitory effect on the production of a spontaneous cancer. Thus, it was also found that the 2% plant fermentation product can act as a prophylactic agent for a spontaneous cancer. In addition, it was also found that SAMP1 can be a model animal for a spontaneous cancer since a cancer was spontaneously produced in SAMP1.

### Test Example 2

### Reduction in time period for production of spontaneous cancer:

In Test Example 1, cancers were spontaneously produced in SAMP1 but the raising time was long. Thus, the reduction in the time period for production of a spontaneous cancer was attempted by adjusting the raising conditions and the like.

Basic raising conditions were the same as in Test Example 1 except that the concentration of the plant fermentation product in the drinking water was 0%, 0.4%, or 2%. Note that 0.4% corresponds to about 2 g/day in a human. In this test, 9 mice were used for the 0% group, 9 mice were for the 0.4% group, and 9 mice were for the 2% group. In addition, 6 young mice (received at 9 weeks old, anatomized at 10 weeks old) were used as controls. The mice were raised in an experimental facility that had a front room (sterilized with a UV light) for barrier between an animal raising room and a corridor and for change of clothes. Such an air conditioning system that gave a difference between the inner pressure of the front room and the inner pressure of the animal raising room to prevent the animal room from contamination was provided. As the raising conditions, for maintaining the SPF conditions, the environment cleaning method was further strengthened. Specifically, wiping of floor with a 500-fold dilution of Hibitane was performed in addition to increasing the frequency of replacement of the outlet filter of the air conditioner (the filter was replaced based on an increase of 1 Pa on the differential pressure gauge in the front room or based on discoloration of the filter due to powder on a floor mat) and installing an air cleaner with plasmacluster. The raising was continued until 72 weeks old.

Table 5 shows the number of mice surviving to 60 weeks old, at which the spontaneous cancer production rate is increased, or longer for each sample group. Note that many of early death examples were considered to be caused by exhaustion due to a masochism action which frequently occurs in solely raising the SAMP1 (male).

**[Table 5]**

| Week-old age at test start (Test Example) | Sample concentration (%) | Tumor bearer % | Total weight of tumors (g) |
|---|---|---|---|
| 12 (1) | 0 | 57.7 | 12.13 |
| | 2 | 16.7 | 2.62 |
| 14 (2) | 0 | 22.2 | 1.47 |
| | 0.4 | 16.7 | 0.71 |
| | 2 | 16.7 | 0.20 |
| 9 (young control) | 0 | 0 | 0 |

In the case of stating at 12 weeks old, the tumor bearer % was apparently smaller in the 2% group and the total weight of tumors was also lower. In the 14 weeks old starting group, not only the tumor bearer % depended on the concentration, but also the total weight of tumors showed an obvious concentration dependency. Most of the tumors produced were found to be B lymphomas as a result of the histopathological determination entrusted to Biopathology Institute, Co., Ltd., and only one example was determined as a hepatocellular adenoma. That is, the inhibitory effect of a plant fermentation product on the production of a spontaneous cancer associated with senescence was apparent.

In the properties of spontaneous cancers produced in SAMP1 (male) mice until 72 weeks old and in the results of histological tissue examinations on the masses, a similar tendency was seen as in the results of Test Example 1, and the masses were mainly lymphomas produced in the mesentery. However, in the produced cancer, not only lymphomas produced in the mesentery but also a liver cancer was observed. This suggests that the cancer production inhibition effect is not limited to for lymphomas.

From the above results, it was found that by raising SAMP1 while strictly maintaining the SPF conditions, the period of time during which a spontaneous cancer is produced can be reduced.

### Test Example 3

### Large intestine aberrant crypt foci (ACF) inhibition test:

Aberrant crypt foci (ACF) are a cluster of abnormal tube-like glands which may be seen on the surface of the lining of the colon and rectum. ACF is formed in an early stage than the formation of colonic polyp, and is one of precancerous lesions (changes that can become a cancer) that are seen in the earliest stage in the colon. Since the present plant fermentation product showed inhibition of the production of tumors, such as lymphomas, spontaneously produced by raising SAMP1 for a long period of time, a study was then conducted about whether the plant fermentation product showed prophylaxis of production of ACF which is a precancerous lesion in the large intestine.

Nine SAMP1 (male) mice per group were solely raised and an aqueous 0% and 2% solutions of a plant fermentation product were prepared in the same manner as in Test Example 1, and were each allowed to be freely taken. Mice of 19 weeks old were used at the start of a test, and the mice were raised for a long period to 73 weeks old in which spontaneous cancer production was observed. As controls, 6 young mice of 9 weeks old were purchased and used from 10 weeks old. The SAMP1 (male) mice were subjected to anesthesia. Then, the large intestine was extracted, was immersed in a 10% formalin solution over night, and was washed with ion exchange water three times in a petri dish, followed by immersion in a 0.02% methylene blue stain solution (ScyTek Laboratories, Inc.). Then, the deeply stained ACF's were counted under a stereoscopic microscope. In the anesthesia, a known mixture of three anesthetic agents was used. ACF's with 4 or more cells/ACF in which the malignancy is higher and ACF's with 4 or less cells/ACF were separately counted (The staining method was determined with reference to "Jukusei Ninniku Chushutsueki niyoru Daicyo Syuyo no Yokusei Koka ni kannsuru Kenkyu (study on the inhibitory effect of matured garlic extract on large intestine tumor) ", JIKIHARA Hiroshi, doctoral dissertation in Hiroshima University (2015), and the determination method was determined with reference to "Daichogan: Shindan to Chiryo no Shinpo, I. Ekigaku to Byotai; 4. Daicyogan no Zengan Jotai (large intestine cancer: progress in diagnosis and therapy, I. epidemiology and clinical conditions; 4. precancerous state of large intestine cancer) " TAKAYAMA Tetsuji, KATSUKI Shinichi, NIITSU Yoshiro, Nihon Naika Gakkai-shi (Journal of The Japanese Society of Internal Medicine, vol. 96, p.24-29 (February 10, 2007)). The results are shown in Table 6.

**[Table 6]**

| Group of mice | Number of mice | Plant fermentation product | ACF (4 or more cells ) /large intestine | ACF (4 or less cells) /large intestine |
|---|---|---|---|---|
| Old | 4 | 0% | 4.3 | 0.0 |
| | 6 | 2% | 1.0 | 0.5 |
| Young | 5 | 0% | 0.8 | 0.6 |

In the case of raising to higher weeks old (73 weeks old) and 0%, the number of the ACF's of 4 or more cells /foci representing high malignancy was 4.3. In the old mice which took the 2% plant fermentation product, the number was 1.0 which was decreased to 1/4 or less and was the same level as in the young mice of 10 weeks old in which no spontaneous cancer was found. The present plant fermentation product showed not only an inhibitory effect on the production of a spontaneous cancer, such as a lymphoma, but also an inhibitory effect on the production in the stage of ACF which is a precancerous lesion.

### Example 1

### Miso-like food:

The plant fermentation product in a paste form produced in Production Example 1 was mixed with Miso and the taste was adjusted with seasonings to produce a Miso-like food.

### Example 2

### Drink:

The plant fermentation product in a paste form produced in Production Example 1 was dissolved in water, which was then mixed with a fructose glucose liquid and a fruit juice, and the taste was adjusted to produce a drink.

### Example 3

### Soft capsule:

The plant fermentation product in a paste form produced in Production Example 1 was mixed with a vegetable oil and lecithin, which was filled in a soft capsule by an ordinary method to produce a soft capsule.

### Example 4

### Chewable tablet:

The plant fermentation product in a paste form produced in Production Example 1 was mixed with crystalline cellulose and white sugar, which was tableted by an ordinary method to produce a chewable tablet.

### Industrial Applicability

According to the present invention, a spontaneous cancer can be prevented. In addition, the spontaneous cancer model animal of the present disclosure, but not part of the invention, can evaluate an effect of a subject substance on a spontaneous cancer.

## Claims

1. A prophylactic agent for use in prophylaxis of a spontaneous cancer, the prophylactic agent comprising, as an active ingredient, a plant fermentation product that is a mixture of (a) to (g):
(a) a koji mold fermentation product of a bean or grain selected from barley, black soybean, red rice, black rice, adzuki bean, adlay, Japanese barnyard millet, foxtail millet, and proso millet;
(b) a yeast fermentation product of a fruit selected from mandarin orange, grape, apple, Vitis Coignetiae berry, peach, persimmon, papaya, Japanese pear, watermelon, Japanese apricot, fig, Chinese quince fruit, kabocha squash, kumquat, Yuzu, loquat fruit, apricot, jujube, chestnut, matatabi fruit, and Japanese plum;
(c) a lactic acid bacterium fermentation product of a root vegetable selected from purple sweet potato, Jerusalem artichoke, carrot, onion, sweet potato, taro, Japanese wild yam, daikon, red turnip, great burdock, lotus root, yacon, lily bulb, arrowhead, ginger, garlic, and turmeric;
(d) a lactic acid bacterium fermentation product of a flower or leafy vegetable selected from cabbage, perilla, Morus leaf, fish mint, Japanese mugwort, Sasa veitchii, and dandelion;
(e) a yeast fermentation product of a seaweed selected from kelp, wakame, and mozuku;
(f) a yeast fermentation product of a seed selected from black sesame, walnut, and ginkgo nut; and
(g) a lactic acid bacterium fermentation product of a mushroom selected from maitake and shiitake,
whereby the plant fermentation product is obtainable or obtained by a process comprising the following steps:
(i) inoculating a culture fluid prepared so as to contain a lactic acid bacterium of *P. acidilacti, L. brevis, L. mesenteroides, L. plantarum, L. lactis, L. sakei,* and *L. casei* at a bacterial concentration of about 1.0 × 10⁵ cfu/g at 0.2% by mass into raw materials of the above (c), (d), and (g), followed by culture at 30°C for 50 hours;
(ii) inoculating a culture fluid prepared so as to contain a yeast of S. *cervisiae* and *Z. rouxii* at a bacterial concentration of about 1.0 × 10⁵ cfu/g at 0.4% by mass into raw materials of the above (b), (e), and (f), followed by culture at 30°C for 50 hours;
(iii) inoculating a koji mold of yellow koji mold, black koji mold, and white koji mold at 0.1% by mass into a raw material of (a), followed by culture at 35°C for 70 hours;
(iv) mixing each culture, followed by culture at 30°C for 200 hours;
(v) subjecting the mash remaining after the fermentation to a solid-liquid separation operation,
(vi) concentrating the resulting filtrate into a paste,
(vii) dispensing the paste into containers, followed by post-fermentation for further one year to obtain the plant fermentation product.

2. The prophylactic agent for use according to claim 1, wherein the plant fermentation product comprises amino acids in the following composition based on 100 g of the plant fermentation product:
| | |
|---|---|
| arginine | 0.2 to 0.6 g |
| lysine | 0.1 to 0.7 g |
| histidine | 0.1 to 0.4 g |
| phenylalanine | 0.2 to 0.8 g |
| tyrosine | 0.1 to 0.6 g |
| leucine | 0.3 to 1.2 g |
| isoleucine | 0.2 to 0.8 g |
| methionine | 0.05 to 0.30 g |
| valine | 0.2 to 0.9 g |
| alanine | 0.2 to 0.9 g |
| glycine | 0.2 to 0.7 g |
| proline | 0.4 to 1.2 g |
| glutamic acid | 1.2 to 3.0 g |
| serine | 0.2 to 0.8 g |
| threonine | 0.2 to 0.7 g |
| aspartic acid | 0.4 to 1.5 g |
| tryptophan | 0.03 to 0.15 g |
| cystine | 0.05 to 0.40 g. |

3. The prophylactic agent for use according to claim 1 or 2, wherein the plant fermentation product comprises organic acids in the following composition based on 100 g of the plant fermentation product:
| | |
|---|---|
| citric acid | 0.5 to 1.2 g |
| malic acid | 0.05 to 0.5 g |
| succinic acid | 0.04 to 0.3 g |
| lactic acid | 0.5 to 6.0 g |
| formic acid | 0.01 to 0.1 g |
| pyruvic acid | 0.005 to 0.05 g |
| free γ-aminobutyric acid | 0.01 to 0.05 g. |

4. The prophylactic agent for use according to any one of claims 1 to 3, further comprising a therapeutic drug for a cancer and/or an alternative therapeutic drug for a cancer.

5. The prophylactic agent for use according to any one of claims 1 to 4, which is in the form of a food or drink.

## Patentansprüche

1. Prophylaktisches Mittel zur Verwendung bei der Prophylaxe von spontan auftretendem Krebs, wobei das prophylaktische Mittel als Wirkstoff ein Pflanzenfermentationsprodukt umfasst, das eine Mischung aus (a) bis (g) ist:
(a) ein Koji-Schimmelpilz-Fermentationsprodukt einer Bohne oder eines Getreides, ausgewählt aus Gerste, schwarzer Sojabohne, rotem Reis, schwarzem Reis, Azukibohne, Hiobsträne, Japanischer Hühnerhirse, Borstenhirse und Rispenhirse;
(b) ein Hefefermentationsprodukt einer Frucht, ausgewählt aus Mandarine, Weintraube, Apfel, *Vitis-coignetiae*-Beere, Pfirsich, Kaki, Papaya, Japanischer Birne, Wassermelone, Japanischer Aprikose, Feige, Chinesischer Quitte, Kabocha-Kürbis, Kumquat, Yuzu, Japanischer Mispel, Aprikose, Jujube, Kastanie, Matatabi-Frucht und Japanischer Pflaume;
(c) ein Milchsäurebakterien-Fermentationsprodukt eines Wurzelgemüses, ausgewählt aus violetter Süßkartoffel, Topinambur, Karotte, Zwiebel, Süßkartoffel, Taro, Japanischer wilder Yamswurzel, Daikon-Rettich, roter Speiserübe, Großer Klette, Lotuswurzel, Yacon, Lilienzwiebel, Pfeilkraut, Ingwer, Knoblauch und Kurkuma;
(d) ein Milchsäurebakterien-Fermentationsprodukt eines Blüten- oder Blattgemüses, ausgewählt aus Kohl, Perilla, Maulbeerblatt, Fischminze, Japanischem Beifuß, *Sasa veitchii* und Löwenzahn;
(e) ein Hefefermentationsprodukt einer Meeresalge, ausgewählt aus Kelp, Wakame und Mozuku;
(f) ein Hefefermentationsprodukt eines Samens, ausgewählt aus schwarzem Sesam, Walnuss und Ginkgonuss; und
(g) ein Milchsäurebakterien-Fermentationsprodukt eines Pilzes, ausgewählt aus Maitake und Shiitake,
wobei das Pflanzenfermentationsprodukt durch ein Verfahren erhältlich oder erhalten ist, das die folgenden Schritte umfasst:
(i) Beimpfen der Rohmaterialien gemäß den obigen Punkten (c), (d) und (g) mit einer Kulturflüssigkeit, die ein Milchsäurebakterium aus der Gruppe *P. acidilacti*, *L. brevis*, *L. mesenteroides*, *L. plantarum*, *L. lactis*, *L. sakei* und *L. casei* in einer Bakterienkonzentration von etwa 1,0 × 10⁵ KBE/g enthält, in einer Menge von 0,2 Massenprozent, gefolgt von einer Kultivierung bei 30 °C für 50 Stunden;
(ii) Beimpfen der Rohmaterialien gemäß den obigen Punkten (b), (e) und (f) mit einer Kulturflüssigkeit, die eine Hefe aus der Gruppe *S. cerevisiae* und *Z. rouxii* in einer Bakterienkonzentration von etwa 1,0 × 10⁵ KBE/g enthält, in einer Menge von 0,4 Massenprozent, gefolgt von einer Kultivierung bei 30 °C für 50 Stunden;
(iii) Beimpfen des Rohmaterials gemäß Punkt (a) mit einem Koji-Schimmelpilz aus der Gruppe Gelber Koji-Schimmelpilz, Schwarzer Koji-Schimmelpilz und Weißer Koji-Schimmelpilz in einer Menge von 0,1 Massenprozent, gefolgt von einer Kultivierung bei 35 °C für 70 Stunden;
(iv) Mischen der jeweiligen Kulturen, gefolgt von einer Kultivierung bei 30 °C für 200 Stunden;
(v) Unterziehen der nach der Fermentation verbleibenden Maische einem Fest-Flüssig-Trennvorgang,
(vi) Aufkonzentrieren des resultierenden Filtrats zu einer Paste,
(vii) Abfüllen der Paste in Behälter, gefolgt von einer Nachfermentation für ein weiteres Jahr, um das Pflanzenfermentationsprodukt zu erhalten.

2. Prophylaktisches Mittel zur Verwendung nach Anspruch 1, wobei das Pflanzenfermentationsprodukt Aminosäuren in der folgenden Zusammensetzung, bezogen auf 100 g des Pflanzenfermentationsprodukts, umfasst:
| | |
|---|---|
| Arginin | 0,2 bis 0,6 g |
| Lysin | 0,1 bis 0,7 g |
| Histidin | 0,1 bis 0,4 g |
| Phenylalanin | 0,2 bis 0,8 g |
| Tyrosin | 0,1 bis 0,6 g |
| Leucin | 0,3 bis 1,2 g |
| Isoleucin | 0,2 bis 0,8 g |
| Methionin | 0,05 bis 0,30 g |
| Valin | 0,2 bis 0,9 g |
| Alanin | 0,2 bis 0,9 g |
| Glycin | 0,2 bis 0,7 g |
| Prolin | 0,4 bis 1,2 g |
| Glutaminsäure | 1,2 bis 3,0 g |
| Serin | 0,2 bis 0,8 g |
| Threonin | 0,2 bis 0,7 g |
| Asparaginsäure | 0,4 bis 1,5 g |
| Tryptophan | 0,03 bis 0,15 g |
| Cystin | 0,05 bis 0,40 g. |

3. Prophylaktisches Mittel zur Verwendung nach Anspruch 1 oder 2, wobei das Pflanzenfermentationsprodukt organische Säuren in der folgenden Zusammensetzung, bezogen auf 100 g des Pflanzenfermentationsprodukts, umfasst:
| | |
|---|---|
| Zitronensäure | 0,5 bis 1,2 g |
| Äpfelsäure | 0,05 bis 0,5 g |
| Bernsteinsäure | 0,04 bis 0,3 g |
| Milchsäure | 0,5 bis 6,0 g |
| Ameisensäure | 0,01 bis 0,1 g |
| Brenztraubensäure | 0,005 bis 0,05 g |
| freie γ-Aminobuttersäure | 0,01 bis 0,05 g. |

4. Prophylaktisches Mittel zur Verwendung nach einem der Ansprüche 1 bis 3, das ferner ein therapeutisches Arzneimittel gegen Krebs und/oder ein alternatives therapeutisches Arzneimittel gegen Krebs umfasst.

5. Prophylaktisches Mittel zur Verwendung nach einem der Ansprüche 1 bis 4, das in Form eines Lebensmittels oder Getränks vorliegt.

## Revendications

1. Agent prophylactique pour une utilisation en prophylaxie d'un cancer spontané, l'agent prophylactique comprenant, comme principe actif, un produit de fermentation végétale qui est un mélange de (a) à (g) :
(a) un produit de fermentation de moisissure de koji d'un haricot ou d'une céréale sélectionné(e) parmi l'orge, le soja noir, le riz rouge, le riz noir, le haricot azuki, la larme-de-Job, le millet japonais, la sétaire d'Italie, et le millet commun ;
(b) un produit de fermentation de levure d'un fruit sélectionné parmi la mandarine, le raisin, la pomme, la baie de Vitis Coignetiae, la pêche, le kaki, la papaye, la poire japonaise, la pastèque, l'abricot japonais, la figue, le coing de Chine, le potiron kabocha, le kumquat, le yuzu, la bibasse, l'abricot, le jujube, la châtaigne, le fruit de matatabi et la prune japonaise ;
(c) un produit de fermentation de bactérie lactique d'un légume racine sélectionné parmi la patate douce violette, le topinambour, la carotte, l'oignon, la patate douce, le taro, l'igname sauvage japonaise, le radis blanc, le navet rouge, la grande bardane, la racine de lotus, la poire de terre, la bulbe de lys, la sagittaire, le gingembre, l'ail et le curcuma ;
(d) un produit de fermentation de bactérie lactique d'une fleur ou d'un légume feuillu sélectionné(e) parmi le chou, la pérille, la feuille de murier, le poivre de chine, l'armoise japonaise, le Sasa veitchii et le pissenlit ;
(e) un produit de fermentation de levure d'une algue sélectionnée parmi les laminaires, le wakame et le mozuku ;
(f) un produit de fermentation de levure d'une graine sélectionné parmi le sésame noir, la noix et la noix de ginkgo ; et
(g) un produit de fermentation de bactérie lactique d'un champignon sélectionné parmi le maitake et le shiitake,
dans lequel le produit de fermentation végétale est apte à être obtenu ou est obtenu par un procédé comprenant les étapes suivantes :
(i) l'inoculation d'un fluide de culture préparé de façon à contenir une bactérie lactique de *P. acidilacti, L. brevis, L. mesenteroides, L. plantarum, L. lactis, L. sakei, et L. casei* à une concentration bactérienne d'environ 1,0 x 10⁵ ufc/g à 0,2 % en masse dans des matières premières des points (c), (d) et (g) ci-dessus, suivie d'une culture à 30 °C pendant 50 heures ;
(ii) l'inoculation d'un fluide de culture préparé de façon à contenir une levure de *S*. *cervisiae et Z. rouxii* à une concentration bactérienne d'environ 1,0 x 10⁵ ufc/g à 0,4 % en masse dans des matières premières des points (b), (e) et (f) ci-dessus, suivie d'une culture à 30 °C pendant 50 heures ;
(iii) l'inoculation d'une moisissure de koji parmi la moisissure de koji jaune, la moisissure de koji noire et la moisissure de koji blanche à 0,1 % en masse dans une matière première de (a), suivie d'une culture à 35 °C pendant 70 heures ;
(iv) le mélange de chaque culture, suivi d'une culture à 30 °C pendant 200 heures ;
(v) la soumission du moût subsistant après la fermentation à une opération de séparation solide-liquide,
(vi) la concentration du filtrat résultant en une pâte,
(vii) la distribution de la pâte dans des contenants, suivie d'une post-fermentation d'un an supplémentaire pour obtenir le produit de fermentation végétale.

2. Agent prophylactique pour une utilisation selon la revendication 1, dans lequel le produit de fermentation végétale comprend des acides aminés dans la composition suivante sur la base de 100 g du produit de fermentation végétale :
| | |
|---|---|
| arginine | 0,2 à 0,6 g |
| lysine | 0,1 à 0,7 g |
| histidine | 0,1 à 0,4 g |
| phenylalanine | 0,2 à 0,8 g |
| tyrosine | 0,1 à 0,6 g |
| leucine | 0,3 à 1,2 g |
| isoleucine | 0,2 à 0,8 g 0,30 |
| méthionine | 0,05 à g |
| valine | 0,2 à 0,9 g |
| alanine | 0,2 à 0,9 g |
| glycine | 0,2 à 0,7 g |
| proline | 0,4 à 1,2 g |
| acide glutamique | 1,2 à 3,0 g |
| sérine | 0,2 à 0,8 g |
| thréonine | 0,2 à 0,7 g |
| acide aspartique | 0,4 à 1,5 g |
| tryptophane | 0,03 à 0,15 g |
| cystine | 0,05 à 0,40 g. |

3. Agent prophylactique pour une utilisation selon la revendication 1 ou 2, dans lequel le produit de fermentation végétale comprend des acides organiques dans la composition suivante sur la base de 100 g du produit de fermentation végétale :
| | |
|---|---|
| acide citrique | 0,5 à 1,2 g |
| acide malique | 0,05 à 0,5 g |
| acide succinique | 0,04 à 0,3 g |
| acide lactique | 0,5 à 6,0 g |
| acide formique | 0,01 à 0,1 g |
| acide pyruvique | 0,005 à 0,05 g |
| acide γ-aminobuty 0,05 g. yrique libre 0,01 à | |

4. Agent prophylactique pour une utilisation selon l'une quelconque des revendications 1 à 3, comprenant en outre un médicament thérapeutique pour un cancer et/ou un médicament thérapeutique alternatif pour un cancer.

5. Agent prophylactique pour une utilisation selon l'une quelconque des revendications 1 à 4, qui se présente sous la forme d'un aliment ou d'une boisson.
